Europäisches Patentamt

European Patent Office

Office européen des brevets

(11) Publication number: **0 445 692 A2**

# EUROPEAN PATENT APPLICATION

(21) Application number: 91103207.6

(51) Int. Cl.5: **C12P 7/64, C11C 3/06**

(22) Date of filing: 04.03.91

(30) Priority: 06.03.90 JP 54715/90

(43) Date of publication of application:
11.09.91 Bulletin 91/37

(84) Designated Contracting States:
BE DE DK ES FR GB GR IT LU NL

(71) Applicant: EUROPEAN ECONOMIC
COMMUNITY E.E.C.
Bâtiment Jean Monnet Plateau du Kirchberg
L-2920 Luxembourg(LU)

(72) Inventor: McNeill, Gerald P.

Ostengasse 4
W-8400 Regensburg(DE)
Inventor: Yamane, Tsuneo
Wakamizu 3-Chome
22-1 Chikusa-ku Nagoya(JP)
Inventor: Shimizu, Shoichi
B-104 Itohpia Higashiyama Garden Heights
3-17-1, Higashiyama Motomachi
Chikusa-ku(JP)

(74) Representative: Weinmiller, Jürgen
Lennéstrasse 9 Postfach 24
W-8133 Feldafing(DE)

(54) A method of producing monoglyceride.

(57) The invention relates to a method of producing monoglyceride. According to the invention, in a process for the synthesis of monoglyceride from fat and oil by its reaction with glycerol using a lipase enzyme as a catalyst, the reaction is carried out in a liquid-liquid-emulsion state and afterwards in a slurry or solid state.

Fig. 1C

This invention relates to a method for producing monoglyceride from fat and oil and glycerol.

Monoglycerides (MG) are widely used as emulsifying agents. In the process of producing MG by means of glycerolysis of fat and oil there known is an oxygen method which improves the quality loss which can be caused by chemical synthesis at high temperature. This oxygen method, which, in comparison with the chemical synthesis, has to be carried out under particular conditions, has the advantage that the quality loss can be reduced, but many by-reactions take place so that the content of MG in the reaction products is low, while the free fatty acid as a by-product requires a raffination step such as fractional distillation etc. When the melting point of fat and oil is higher than the reaction temperature, it is necessary to dissolve fat and oil in a solvent. In spite of this step, the content of MG in the reaction products is low and ranges from 20 to 30% containing diglyceride in a range from 40 to 60%. According to the conventional method it was impossible to obtain reaction products with a high MG content.

In order to eliminate these defects, this invention refers to a new method for producing monoglyceride of a high yield by the reaction of fat and oil and glycerol using a lipase enzyme wherein the content of undesired by-products is very low and MG is obtained with a high yield.

This problem is solved by carrying out the reaction using lipase firstly in a liquid-liquid-emulsion state and subsequently in a slurry or solid state so that, without dissolving fat and oil in a solvent, monoglyceride can be obtained with a high yield.

Now, the process according to this invention will be explained in detail.

In the frame of the invention, fat and oil can be of animal or vegetable or microbial origin, but this invention can have a great effect especially in application to palm oil, palm olein, palm stearin, pig fat, tallow, milk fat, cocoanut fat, which according to the conventional oxygen method because of their high melting point needed to be dissolved in a solvent. The water content in the reaction system ranges preferably from 0,5 to 10% related to glycerol. Under 0,5%, the reaction is low, while above 10%, free fatty acid can be mixed as a by-product.

The ratio between fat or oil and glycerol as raw materials ranges advantageously between 1:1 and 1:5. A small amount of glycerol results in a small quantity of monoglyceride. If too much glycerol is used, the unconverted part of glycerol remains the product and influences the efficiency negatively.

The use of lipase results in a high conversion rate of fat and oil and in a high yield of monogylceride. Among the different lipase species, Pseudomonas fluorescens, Chromobacterium viscosum, Pseudomonas fragi, Pseudomonas cepacia, can be used as free lipase, but also as solid lipase.

The optimal temperature which is necessary to obtain the highest yield of monoglyceride varies in accordance with the particular fat and oil as raw material and is usually near the melting point of each fat or oil.

The reaction is carried out under stirring at the optimal temperature near the melting point of fat and oil, but it is necessary that in the first phase the reaction takes place in a liquid-liquid-emulsion state. If the optimal temperature is lower than the melting point of fat and oil, the reaction system must be put into a liquid-liquid-emulsion state or heated. When heating is to be carried out, the temperature has to be lowered afterwards to the optimal temperature. While keeping the optimal temperature near the melting point of fat and oil and continuously stirring, the reaction goes on and monoglyceride can be obtained. Finally, the reaction system becomes a slurry or solid and further stirring is impossible but the reaction is allowed to continue.

As a result of the above described process, the reaction products with a monoglyceride content of more than 70% can be obtained.

According to this invention, even if the optimal temperature of lipase is lower than the melting point of fat and oil, the enzymatic reaction is possible without dissolving fat and oil in a solvent to obtain monoglyceride of high yield (over 70%).

The invention will now be explained with reference to examples and the drawings.

Figures 1a, 1b, 1c, show the effect of the reaction temperature on the glyceride yield, the time being plotted on the abscissa in hours. While the temperature is fixed to 50°C and 40°C in Figures 1a and 1b respectively, the case of Figure 1c implies a temperature change from 50°C to 40°C after an initial period of 20 hours.

Figure 1d shows the monoglyceride yield as a function of temperature for tallow.

Figure 1e shows the yield of monoglyceride production from tallow as a function of time (in hours) for various reaction temperatures.

Figures 2a and 2b explain the effect of the water content (A to E) on the glycerolysis of tallow.

Figure 3 refers to the influence of the water content A to E on the glycerolysis of palm oil.

Figure 4 shows the effect of the mol ratio A to E between glycerol and tallow.

In Figure 5, palm oil (dashed lines) and beef tallow (continued lines) are compared as far as

EP 0 445 692 A2

monoglyceride formation is concerned (reaction at 40°C).

By the way, in the figures, MG means monoglyceride, DG means diglyceride, TG means triglyceride and FFA means free fatty acid.

The enzyme-catalyzed glycerolysis of fat and oil in the examples and measurements of fat or oil composition and water content are carried out according to following methods.

An appropriate amount of distilled water was dissolved in 2,7g glycerol (purchased from WAKKI ZYUNYAKU - Wako Pure Chemical Ind.) to give a final content of 3% water in glycerol. Lipase (approximately 7000 units) was added to the solution and 13g of fat was dissolved. This resulted in a mole ratio of glycerol to fat of 2:1. After arriving at the reaction temperature a mixture of glycerol and enzyme was added. The reaction was carried out under constant temperature condition in a water bath and stirring with 800 rpm. One lipase activity is defined as the amount of enzyme which liberates under the given reaction condition 1 $\mu$mol free fatty acid per minute (J.Jpn.Oil Chem.Soc., Vol. 36, N° 9, 638-642(1987)). The analysis of the fat composition in the product mixture was carried out according to the Itatroscan method (J.Jpn.Oil Chem.Soc., Vol. 35, N° 8, 625-631 (1986)). Water contents were determined in accordance with the Karl-Fischer titration method (J.Jpn.Oll Chem.Soc. Vol. 35, N° 8, 625-631 (1986)).

Example 1 Effect of temperature

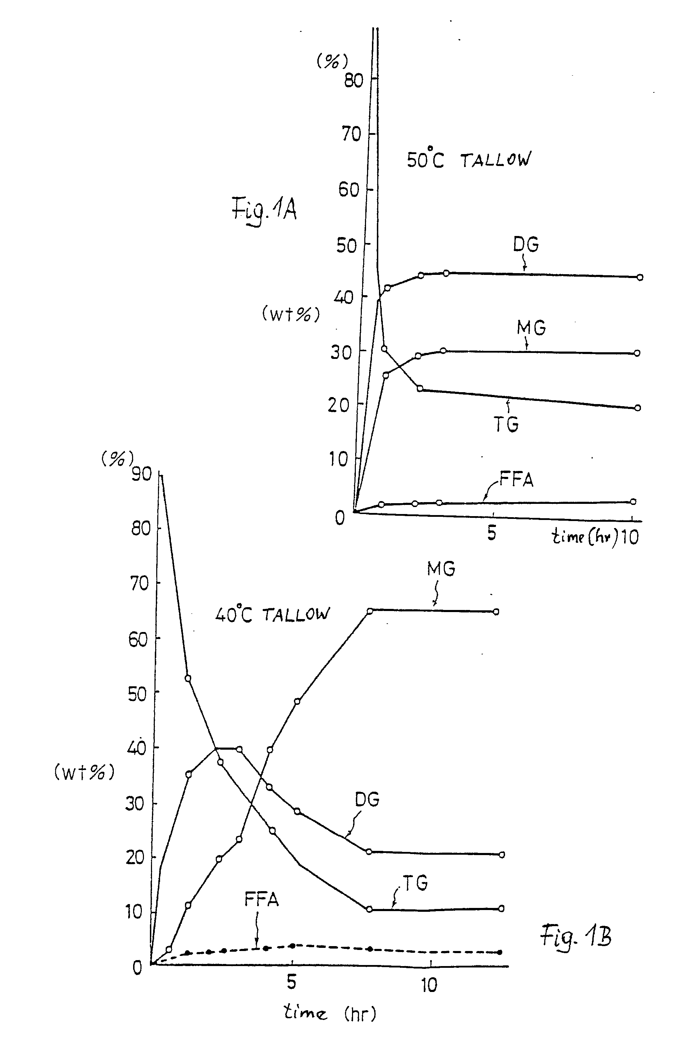

Glycerolysis of tallow as fat (melting point 45-48°C; purchased from Nippon Yushi) was carried out using lipase obtained from Pseudomonas fluorescens (AMANO SEIYAKU) at a reaction temperature of 50°C. The other conditions were the same as in the above-described method. The composition of the reaction mixture was analyzed in the course of time and this result is shown in Figure 1a. After 8 hours the analytical values were triglyceride 22%, diglyceride 46%, monoglyceride 31% and free fatty acid 4%. The reaction was continued under agitation by stirring in liquid-liquid-emulsion state at 40°C.Figure 1b shows that after 4 hours the reaction mixture became solid while the analysis of its composition showed triglyceride 10%, diglyceride 22%, monoglyceride 65% and free fatty acid 4%. From this observation it can be concluded that the reaction by keeping the reaction temperature under the melting point results in about two times higher yield of monoglyceride. Figure 1c shows the analysis result when the reaction started at 50°C, and after equilibrium state the temperature was lowered to 40°C. This result is in accordance with Figures 1a and 1b.

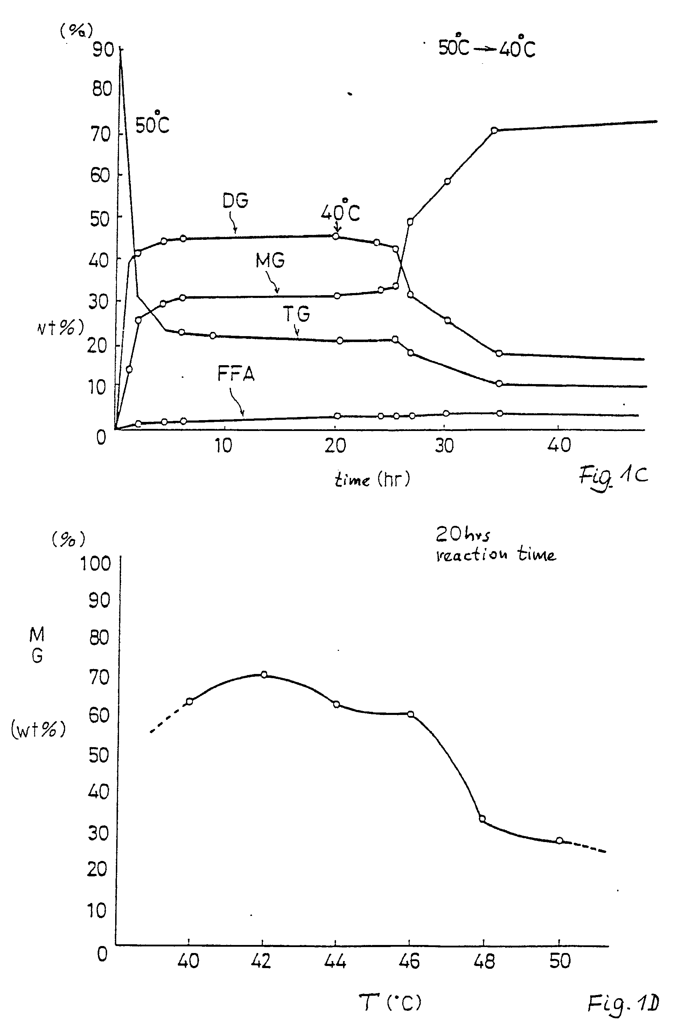

Next, the reaction was changed step by step from 40°C to 50°C to investigate the monoglyceride content after reaction time of 20 hours. The result is shown in Figure 1d. Figure 1e shows the relation between reaction temperature and reaction time. This observation confirms that by keeping the reaction temperature under the melting point the monoglyceride yield increases rapidly. Since the optimal temperature for tallow is 42°C, the following tests with tallow will be carried out only at 42°C.

Example 2 Effect of water content

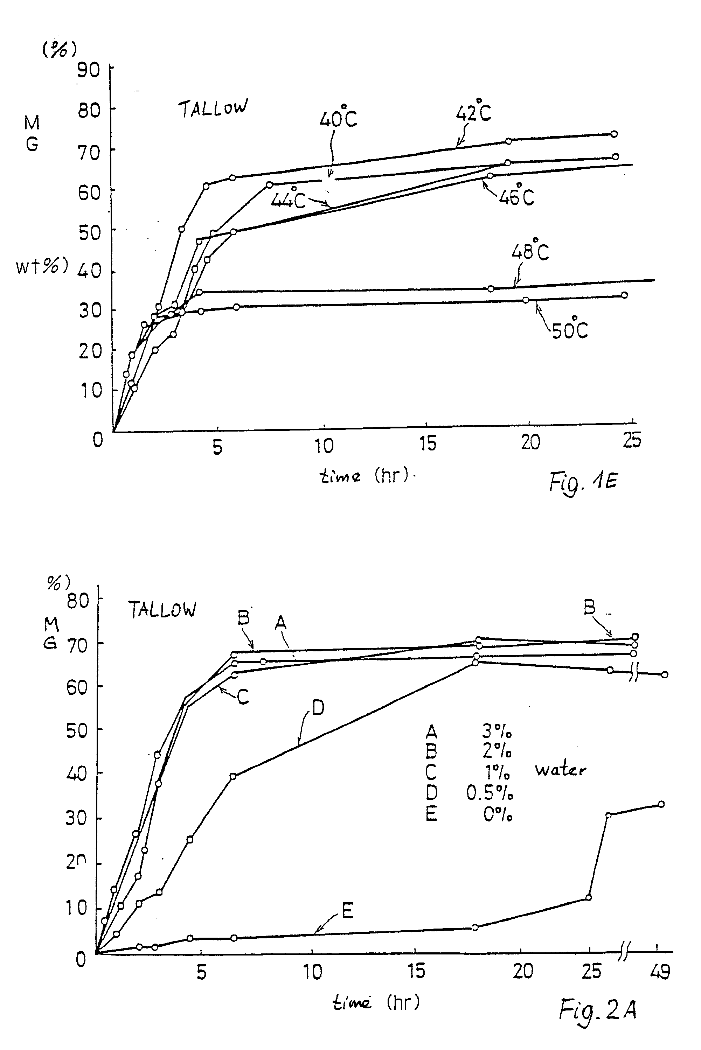

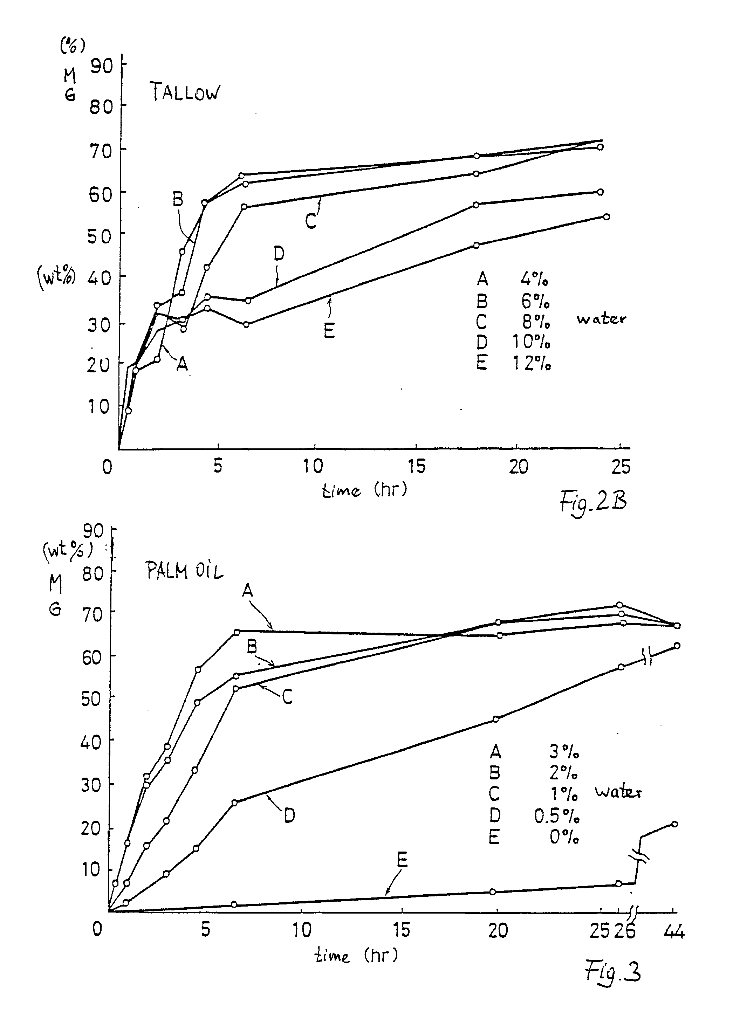

Figure 2 shows the monoglyceride production in the reaction mixture with a water content in the glycerol phase ranging from 0 to 12% during glycerolysis of tallow at 42°C under agitation or stirring while the other conditions were the same as in example 1. From this it follows that in the range of a water content from 8 to 12% at the time when the monoglyceride content reached about 30% the reaction rate became slow. Figure 2a shows that with a water content cf 0% (curve E), the monoglyceride content is low. The initial rates during the reaction under the above-mentioned condition and the formation of FFA (free fatty acid) and monoglyceride after glycerolysis are shown in table 1. From this it follows that in the range of low water content (0,6 to 3,7%) the water content has a great effect on the initial rate and with increasing water content the formation of free fatty acid as by-product increases while the monoglyceride formation is going down. Figure 3 shows the result of glycerolysis of palm oil instead of tallow under the identical conditions. In the case of glycerolysis of palm oil, a relatively low water content results in an increasing monoglyceride formation. From the above-mentioned results it can be concluded that a water content ranging from 0,5 to 10%, better from 0,5 to 3%, means the glycerolysis condition under which a high yield of monoglyceride can be obtained.

3

Table 1

Effect on water content

| water content in glycerol (%) | initial rate* (%/hr) | free fatty acid after reaction (%) | monoglyceride after reaction (%) |
|---|---|---|---|
| 12,3 | 21,4 | 8,6 | 56 |
| 10,3 | 19,2 | 6,0 | 62 |
| 8,5 | 20,1 | 4,9 | 68 |
| 6,3 | 20,2 | 3,5 | 73 |
| 4,6 | 16,2 | 3,0 | 69 |
| 3,7 | 13,4 | 2,5 | 69 |
| 2,5 | 9,1 | 1,9 | 74 |
| 1,8 | 11,5 | 1,3 | 73 |
| 1,2 | 4,7 | 0,9 | 72 |
| 0,6 | 1,1 | 0,3 | 39 |

*Initial rate has to be understood as the rate from the very start of glycerolysis to some hours after that.

The following examples were carried out with a fixed water content of 3%.

Example 3 Effect of lipase enzyme

When using lipases obtained from Chromobacterium viscosum (purchased from TOYO JOZO), Psuedomonas fluorescens (AMANO SEIYAKU), Mucor miehei (NOVO IND. Japan), SP398 (NOVO IND. Japan), fixed lipase obtained from Lipozyme, Mucor miehei (NOVO IND. Japan) and lipase obtained from Rhizo-pus japonicus (Osaka bacteria reseach center) and under the same conditions as example 2 (temperature 42°) the reactions were carried out. The results of the analysis of the equilibrium composition of the reaction mixture are shown in table 2. From this it can be shown that by glycerolysis using lipases obtained from Chromobacterium viscosum and Pseudomonas fluorescens a high yield of monoglyceride can be achieved while the triglyceride content is low and the formation of diglyceride and free fatty acid as by-products is small.

In the following examples, lipase obtained from Pseudomonas fluorescens (crude enzyme) was used.

Table 2

Effect of the lipase type on monoglyceride
after glycerolysis of tallow

| Lipases (obtained from) | fat composition (%) | | | | |
|---|---|---|---|---|---|
| | triglyce-ride | 1,3-digly-ceride | 1,2-digly-ceride | monogly-ceride | free fatty acid |
| Chromobacterium viscosum | 8 | 11 | 5 | 72 | 3 |
| Pseudomonas fluorescens (crude enzyme) | 8 | 11 | 4 | 72 | 3 |
| Pseudomonas fluorescens (refined enzyme) | 8 | 13 | 5 | 70 | 3 |
| Mucor miehei | 13 | 10 | 13 | 59 | 4 |
| SP398 | 21 | 13 | 16 | 45 | 5 |
| Lipozyme (fixed en-zyme of lipase ob-tained from Mucor miehei) | 45 | 10 | 16 | 25 | 4 |

Example 4 Effect of glycerol content

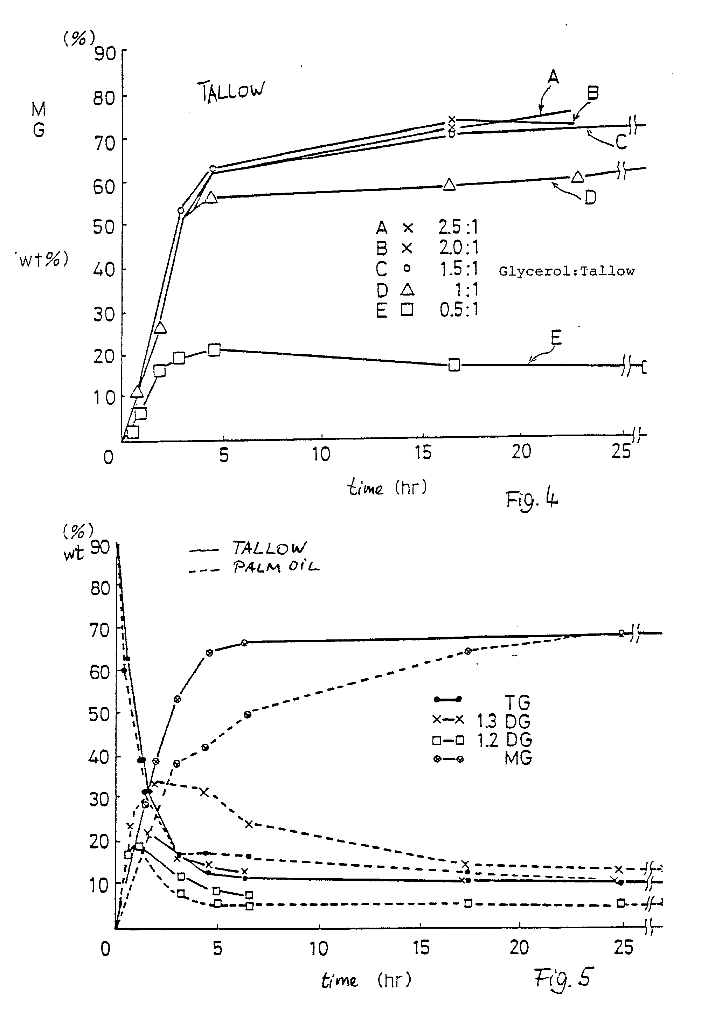

Figure 4 shows the initial rate of the formation of monoglyceride in the reaction mixture during glycerolysis of beef tallow with a mol ratio of beef tallow to glycerol ranging from 1:0,5 to 1:2,5 and under the same conditions as Example 1. The reaction was carried out first at 42°C and under agitation of stirring. The analysis shows that the range of mol ratio 1:1 to 1:2,5 results in a high production of monoglyceride. This observation is exactly in accordance with the fact that in the mol ratio range of fat:glycerol of 1:2 the glycerolysis can take place stoechiometrically.

Example 5 Effect of fat and oil

Instead of beef tallow, milk fat (purchased from MORINAGA NYUGYO), palm oil (FUJI SEIYU), palm olein (FUJI SEIYU), palm stearin (FUJI SEIYU), pig fat (NIPPON YUSHI), cocoanut oil (AJINOMOTO) and rapeseed oil (AJINOMOTO) were used to carry out glycerolysis at different reaction temperatures according to fat and oil while the other reaction conditions were the same as in example 1. At each optimal reaction temperature the reaction was carried out first in a liquid-liquid-state and further in a slurry state or solid state in order to investigate the effect of fat and oil on the highest yield of monoglyceride (table 3). By using glycerolysis of milk fat, palm oil, palm olein, palm stearin, pig fat and cocoanut oil a high monoglyceride yield ranging from 70 to 80% can be achieved.

Concerning the glycerolysis of rapeseed oil the reaction time is about five times as long as with other fats and oils because the melting point of rapeseed oil is considerably lower than the one of other fats and oils.

From this it follows that palm oil which is relatively cheap and is used widely in many industrial fields, especially palm stearin, results in the highest yields of monoglyceride. This means that the process according to this invention is very useful.

<u>Table 3</u>

Effects of different fats and oils

| fat and oil type | melting point (°C) | optimal tempe-rature (°C) | reaction time (hr) | equilibrium composition | | | |
|---|---|---|---|---|---|---|---|
| | | | | trigly-ceride | digly-ceride | mono-glyce-ride | free fatty acid |
| beef tallow | 45 to 48 | 42 | 24 | 8 | 13 | 76 | 3 |
| milk fat | 28 to 38 | 32 | 24 | 1 | 15 | 80 | 4 |
| palm oil | 31 to 40 | 40 | 24 | 7 | 13 | 77 | 3 |
| palm olein | 20 to 24 | 30 | 24 | 10 | 12 | 71 | 7 |
| palm stearin | 44 to 56 | 40 | 20 | 4 | 10 | 86 | 0 |
| pig fat | 28 to 48 | 30 | 14 | 10 | 15 | 70 | 5 |
| cocoanut oil | 24 to 28 | 30 | 48 | 3 | 18 | 77 | 2 |
| rapeseed oil | 0 to 12 | 5 | 120 | 10 | 11 | 77 | 2 |

Unless otherwise stated, beef tallow as fat and lipase, which was obtained from Pseudomonas fluorescens (crude enzyme), were used in all examples referring to figures. The reaction is based on a water content of 3% related to glycerol, a mol ratio of glycerol: fat of 2:1 and each optimal temperature as reaction temperature (table 3). All data without compound name refer to monoglyceride content.

According to the method to which the invention refers, the conversion rate can be improved and the formation of diglyceride and free fatty acid as by-products can be reduced. This results in the advantage of producing monoglyceride as emulsifying agents of high quality which can be obtained from cheap crude materials and with a high yield, but without quality loss. The invention provides an industrial producing method of great usefulness.

## Claims

1. A method of producing monoglyceride, characterized in that in a process for the synthesis of monoglyceride from fat and oil by its reaction with glycerol using a lipase enzyme as a catalyst the reaction is carried out in a liquid-liquid-emulsion state and afterwards in a slurry or solid state.

2. A method according to claim 1, characterized in that the reaction system with lipase enzyme has a water content in the range of 0,5 to 10 wt% related to glycerol.

Fig. 1A

50°C TALLOW

(%)

(wt%)

DG

MG

TG

FFA

time (hr) 10

(%)

(wt%)

40°C TALLOW

MG

DG

TG

FFA

Fig. 1B

time (hr)

7

Fig. 1C

Fig. 1D

Fig. 1E

| | | |
|---|---|---|
| A | 3% | |
| B | 2% | |
| C | 1% | water |
| D | 0.5% | |
| E | 0% | |

Fig. 2A

Fig.2B

Fig.3

Fig. 4

Fig. 5